Europäisches Patentamt

(19) European Patent Office

Office européen des brevets

(11) Veröffentlichungsnummer: 0 079 528
A1

## (12) EUROPÄISCHE PATENTANMELDUNG

(21) Anmeldenummer: 82110151.6

(22) Anmeldetag: 04.11.82

(51) Int. Cl.³: **C 12 N 5/02**
**C 12 Q 1/24**

(30) Priorität: 06.11.81 DE 3144081

(43) Veröffentlichungstag der Anmeldung:
25.05.83 Patentblatt 83/21

(84) Benannte Vertragsstaaten:
AT BE CH DE FR GB IT LI NL SE

(71) Anmelder: HOECHST AKTIENGESELLSCHAFT
Postfach 80 03 20
D-6230 Frankfurt/Main 80(DE)

(72) Erfinder: Betz, Joachim, Dr.
Hohe Stätte 16
D-6000 Frankfurt am Main 56(DE)

(72) Erfinder: Leineweber, Michael, Dr.
Sachsenring 10
D-6238 Hofheim am Taunus(DE)

(72) Erfinder: Mracek, Miroslav
Bornwiesenweg 67
D-6000 Frankfurt am Main 1(DE)

(54) Automatisiertes Verfahren zur Selektion von Zellklonen für Submerskulturen.

(57) Automatisiertes Verfahren zur Selektion von Zellklonen für Submerskulturen, bei dem man zur Erhöhung der Anzahl der pro Zeiteinheit auszuprüfenden Zellklone mehrere an sich bekannte Einzelvorrichtungen gemeinsam einsetzt, wobei

a) sterile Mikrotiterplatten unter sterilen Bedingungen automatisch mit Nährlösungen beschickt werden;

b) jede Kavität der Mikrotiterplatte automatisch mit je einer Zelle beimpft wird;

c) die beimpften Mikrotiterplatten stapelweise in einem Schüttelinkubator unter Submerskulturbedingungen bebrütet werden;

d) ein Filtrat der Nährlösung oder ein Extrakt der Zellkultur hergestellt und hierin nach einer an sich bekannten Methode die Menge des gewünschten Stoffwechselproduktes gemessen wird.

Croydon Printing Company Ltd

Automatisiertes Verfahren zur Selektion von Zellklonen für Submerskulturen

Gegenstand der Erfindung ist ein automatisiertes Verfahren zur Selektion von Zellklonen für Submerskulturen,
mit dem die Aufgabe gelöst wird, eine größere Anzahl
von Zellklonen in kürzerer Zeit auszutesten, als es
mit den bisher bekannten Methoden möglich war. Hierdurch
wird die Chance erheblich erhöht, spezielle Mutanten
zu finden, die unter submersen Bedingungen entweder
das gewünschte Stoffwechselprodukt überhaupt oder in
höherer Ausbeute bilden können. Zur Lösung dieser Aufgabe werden mehrere, an sich bekannte Einzelvorrichtungen
in einer neuen und sinnvollen Weise miteinander kombiniert, wodurch ein automatisierter Verfahrensablauf
erreicht wird.

Die Verbesserung der Eigenschaften von Zellklonen ist für
die industrielle Mikrobiologie eine ganz entscheidende
Aufgabe. Sobald ein zellulärer Organismus gefunden ist,
der ein gesuchtes Stoffwechselprodukt produziert oder
eine bestimmte Reaktion katalysiert, kommt es darauf
an, leistungsfähigere Zellklone zu finden, die eine
höhere Ausbeute des gewünschten Produktes liefern. Nur
auf diesem Wege können Antibiotika, Cytostatika, Enzyme,
Enzyminhibitoren, Hormone und andere Wirkstoffe in
Mengen gewonnen werden, die eine industrielle Herstellung erlauben.

Zur Verbesserung der Ausbeuten an Stoffwechselprodukten
von pro- als auch von eukaryotischen Zellen versucht
man, das genetische Material der betreffenden Zelle
in der gewünschten Richtung zu verändern. Dies kann
durch Bestrahlung oder durch mutagene Chemikalien, neuerdings aber auch mittels Kernfusionierung oder durch Maßnahmen erfolgen, die heute unter dem Begriff "genetic engineering" zusammengefaßt werden. Nach Durchführung eines derartigen Versuchs zur Änderung des genetischen Materials liegen aber naturgemäß die unveränderten Ausgangsindividuen in weit höherer Zahl vor als diejenigen zellulären Organismen, deren Stoffwechselleistung verbessert werden konnte oder die jetzt sogar erstmalig einen neuen wertvollen Stoff produzieren.

Die Isolierung einer durch derartige Methoden hergestellten Mutante mit verbesserten Eigenschaften erfolgt auf an sich bekannten Wegen. Zunächst muß die die gewünschte Mutante enthaltende Population soweit verdünnt werden, daß statistisch in einem Aliquot nur noch eine einzige Zelle enthalten ist, die nach Abtrennung und mehrfacher Zellteilung unter geeigneten Kulturbedingungen zu einem Klon führt. Die Identifizierung der gewünschten Mutante kann, wenn es um die Ausbeutesteigerung eines bekannten Antibiotikums geht, entweder durch eine Analyse der Flüssigkeitskulturen oder durch Messung der antibiotischen Aktivität gegen bestimmte Teststämme erfolgen. Besonders bewährt hat sich dabei die von Ichikawa und Mitarbeitern (T.Ichikawa, M.Pate und A.Ozak in "Folia Microbiologica" 16, 218-224 (1971)) eingeführte Agar-Stück-Methode. Bei dieser Methode produzieren die aus Einzelzellen hervorgegangenen Klone auf einen mit Nährstoffen angereicherten Agar-Blöckchen das Antibiotikum, welches sich in diesem Agar-Blöckchen anreichert. Setzt man dieses Agar-Blöckchen nun auf ein halbfestes Kulturmedium mit einem Testorganismus, so wird dieser in Abhängigkeit von der Antibiotika-Konzentration, die aus dem Agar-Blöckchen hinaus diffundiert, gehemmt oder zum Absterben gebracht.

Überraschenderweise haben sich jedoch die nach dieser Methode isolierten Zellklone trotz hervorragender Testergebnisse bei der Agar-Stück-Methode in großtechnischen Produktionen oft nicht sonderlich bewährt. Dieses Phänomen wird dadurch erklärt, daß großtechnische, mikrobiologische Produktionen in Fermentern als Submerskulturen durchgeführt werden, also unter Bedingungen, die sich deutlich von den Selektionsbedingungen unterscheiden, die zum Beispiel bei der Agar-Stück-Methode angewendet werden.

Es stellte sich deshalb die Aufgabe, schon bei der Selektion von Zellklonen im Labormaßstab Bedingungen anzuwenden, die denen einer großtechnischen Submers-Kultur im Fermenter entsprechen.

Die Erfindung betrifft deshalb ein Verfahren zur Selektion von Zellklonen für Submerskulturen, wobei man Zellklone in Submersnährböden impft, kultiviert und auswertet, das dadurch gekennzeichnet ist, daß man in einem automatisierten Verfahren als Kulturgefäße die Kavitäten der Mikrotiterplatten einsetzt und jede Kavität der Mikrotiterplatte mit je einer Zelle beimpft.

Bei diesem Verfahren wird zunächst unter sterilen Bedingungen ein steriles Nährmedium in sterile Mikrotiterplatten dispensiert. Die Nährlösung muß eine geeignete Kohlenstoffquelle, z.B. Glukose in einer Menge zwischen 1 und 10 Gew.-%, vorzugsweise etwa 2,5 Gew.-%, enthalten. Eine geeignete Stickstoffquelle, z.B. Peptone, Bouillon, Fleischextrakt oder Ammoniumsalze, soll in einer Menge zwischen 0,5 und 5 Gew.-%, vorzugsweise in einer Menge von etwa 1 Gew.-%, vorhanden sein. Außerdem werden zweckmäßigerweise auch noch andere einfache oder komplexe Nährstoffe wie Proteine, Seren, Mineralsalze,

0079528

Spurenelemente und Vitamine in üblicher Weise der Nährlösung zugesetzt. Die Mikrotiterplatten werden dann mit dieser Nährlösung, nachdem sie in einem Vorratsbehälter der Dispensiermaschine ebenso wie deren Schlauchleitungen und Dispensierkopf sterilisiert worden ist, beschickt.

Die gefüllten Mikrotiterplatten werden dann nach Auflegen eines sterilen Deckels in einer Transport- und Stapelbox automatisch zum Impfgenerator transportiert. Im sterilen Innenraum des Impfgenerators werden die Mikrotiterplatten automatisch auf einen x-y-Abtast-Tisch gesetzt. Dieser x-y-Abtast-Tisch führt die Mikrotiterplatte mäanderförmig durch den Impfgenerator, wobei in jede Kavität der Mikrotiterplatte eine in einen Mikrotropfen eingehüllte Impfzelle eindosiert wird. Als Impfgeneratoren haben sich dabei Durchflußcytofotometer bewährt, wie sie die Firmen Ortho Instruments, Becton-Dickinson oder Coulter Electronics herstellen. Mit derartigen Instrumenten können auch keimfähige von abgestorbenen Zellen unterschieden und letztere verworfen werden.

Nach der automatischen Beimpfung wird die Mikrotiterplatte wieder abgedeckt und automatisch zu ihrer ursprünglichen Stelle in der Transport- und Stapelbox zurücktransportiert. Eine hierfür besonders geeignete Stapelbox für Mikrotiterplatten ist in der deutschen
Gebrauchsmusteranmeldung G 81 29 742.4
beschrieben.

Mehrere mit Mikrotiterplatten gefüllte Stapelboxen
werden dann bei konstanter Temperatur und hoher Luftfeuchtigkeit in einem Schüttelinkubator bebrütet,bis
die gewünschte Zelldichte eingetreten ist, wozu 1
bis 5 Tage erforderlich sind.

Wichtig ist nun, daß bei der Bebrütung im Schüttelinkubator die Submers-Bedingungen möglichst weitgehend
simuliert werden. Dieses Ziel wird in vollkommener Weise
dann erreicht, wenn die Stapel der Mikrotiterplatten
mit einer Amplitude zwischen 0,2 und 7 cm, vorzugsweise
mit einer Amplitude von etwa 5 cm,und mit einer Schüttelfrequenz von 100 bis 400 Upm, vorzugsweise mit einer
Schüttelfrequenz von 250 Upm, bei einer Temperatur
zwischen 20 und 40°C, vorzugsweise bei etwa 30°C, und
bei einer relativen Luftfeuchte von 60 bis 95 %, vorzugsweise von 90 %, geschüttelt werden.

Von jeder einzelnen Kultur, die unter den genannten
Bedingungen auf den als Vorkulturplatten zu bezeichnenden
Mikrotiterplatten hergestellt worden ist, wird anschließend unter sterilen Bedingungen auf einer zweiten
als Hauptkulturplatte anzusprechenden weiteren Mikrotiterplatte, die mit dem gleichen Nährmedium beschickt
ist, durch Überimpfen von Zellen mittels eines automatisch arbeitenden Inokulators eine entsprechende
Tochterkultur hergestellt. Die Bebrütung der Hauptkulturplatten erfolgt dabei im Schüttelinkubator in vollkommen gleicher Weise wie die der Vorkulturplatten.

Die Vorkulturplatten werden anschließend im Kühlraum
als Quelle für den gesuchten neuen und verbesserten
Stamm aufbewahrt, bis die Ergebnisse der mit den Haupt-

kulturplatten durchgeführten Tests vorliegen.

In der Regel ist das gesuchte Produkt ein Stoffwechselprodukt, das in das Nährmedium abgegeben wird. Es genügt dann, das Filtrat des Nährmediums auf das Vorhandensein und die Menge der gesuchten Substanz zu testen. Handelt es sich jedoch bei den gesuchten Stoffen um zellgebundene Stoffe, dann ist ein Aufschluß der Zellen notwendig. Für diesen Aufschluß stehen eine Reihe bekannter Verfahren zur Verfügung. In jedem Fall ist also zunächst eine Sedimentation der Zellen vorzunehmen, wobei die zu testende Lösung im ersten Fall der Überstand, im zweiten Fall der Zellextrakt ist, der nach Verwerfen des Überstandes, Zellaufschluß und Abtrennen der Zellreste gewonnen wird.

Selbstverständlich hängt es von der Natur des gesuchten Stoffwechselproduktes ab, welche Nachweismethode zum Einsatz kommt. Für die hier notwendigen Reihenuntersuchungen ist es jedoch besonders empfehlenswert, mit dem Enzym-immuno-sorbent-Test (= ELISA) zu arbeiten. Hierbei wird zunächst in an sich bekannter Weise der gegen den gesuchten Stoff gerichtete Antikörper hergestellt und mit diesem Antikörper die Wand der Mikrotiterplattenkavitäten maschinell beschichtet. Zu einer derartig vorbereiteten Mikrotiterplatte wird dann mittels einer automatisch arbeitenden Pipettiermaschine ein Aliquot der zu testenden Lösung aus der Hauptkulturplatte oder einer daraus hergestellten Verdünnung zugegeben. Mit einer weiteren Pipettiermaschine wird dann ein mit einem Enzym markiertes kompetitierendes Agens zugefügt, welches um die begrenzten Antikörperbindungsstellen an der Wand in Konkurrenz tritt. Nach dem automatischen Auswaschen aller Kavitäten wird mittels eines automatischen Dispensers ein Enzymsubstrat zugegeben, welches von den gebundenen Antigen-Enzymkomplexen zu einem farbigen Produkt umgesetzt wird. Durch automatische Zugabe einer Stoplösung wird die Reaktion beendet und die Platte kann dann automatisch, z.B. von einem Spektralphotometer, der die Mikrotiterplatte reihenweise abtastet, in ihrer optischen Dichte vermessen werden. Handelt es sich bei dem gesuchten Stoffwechselprodukt um ein Enzym oder einen Enzyminhibitor, dann vereinfacht sich der Test, weil man direkt durch Zugabe eines Substrats oder durch die Zugabe von Enzym und Substrat die Reaktion spektrophotometrisch erfassen kann.

Gelegentlich lassen sich mit dem gesuchten Stoffwechselprodukt auch Hemmteste durchführen, bei denen der produzierte Stoff zur Verhinderung oder Verlangsamung des Wachstums von zugegebenen Testorganismen führt. Die dadurch hervorgerufenen Änderungen der optischen Dichte können ebenfalls mit einem Spektralphotometer, der die Mikrotiterplatten reihenweise abtasten und in ihrer optischen Dichte vermessen kann, erkannt werden.

Alle so gewonnenen Testwerte werden in einen Computer eingelesen, der anhand einer Eichkurve und mit Hilfe von täglich durchgeführten Standardmessungen die Aktivität jedes Klons klassifiziert. Überdurchschnittlich produzierende Zellklone können so ohne weiteres identifiziert und aufgefunden werden.

Anschließend erfolgt eine Überprüfung durch steriles Abimpfen aus der gekühlt gehaltenen Vorkulturplatte mittels üblicher mikrobiologischer und zellbiologischer Arbeitstechniken.

PATENTANSPRÜCHE:

1. Verfahren zur Selektion von Zellklonen für Submerskulturen, wobei man Zellklone in Submersnährböden impft,
kultiviert und auswertet, dadurch gekennzeichnet, daß
man in einem automatisierten Verfahren als Kulturgefäße
die Kavitäten der Mikrotiterplatten einsetzt und jede
Kavität der Mikrotiterplatte mit je einer Zelle beimpft.

2. Verfahren nach Anspruch 1, dadurch gekennzeichnet, daß

a) sterile Mikrotiterplatten unter sterilen Bedingungen
automatisch mit Nährlösung beschickt werden;

b) jede Kavität der Mikrotiterplatte automatisch mit
je einer Zelle beimpft wird;

c) die beimpften Mikrotiterplatten stapelweise ·in einem
Schüttelinkubator unter Submerskulturbedingungen
bebrütet werden;

d) ein Filtrat der Nährlösung oder ein Extrakt der
Zellkultur hergestellt und hierin nach einer
an sich bekannten Methode die Menge des gewünschten
Stoffwechselproduktes gemessen wird.

3. Verfahren nach Anspruch 1 und 2, dadurch gekennzeichnet,
daß die Mikrotiterplatten mit einer Nährlösung
beschickt werden, welche in einem Vorratsbehälter
der Dispensiermaschine ebenso wie deren Schlauchleitungen und der Dispensierkopf sterilisiert worden
ist.

4. Verfahren nach einem oder mehreren der Ansprüche 1 bis
3, dadurch gekennzeichnet, daß die Kavitäten der Mikrotiterplatte durch einen Impfgenerator mit jeweils einer
keimfähigen Zelle des Organismus beimpft werden.

5. Verfahren nach einem oder mehreren der Ansprüche 1 bis 4, dadurch gekennzeichnet, daß die beimpften Mikrotiterplatten 1 bis 5 Tage im Schüttelinkubator bebrütet werden.

6. Verfahren nach einem oder mehreren der Ansprüche 1 bis 5, dadurch gekennzeichnet, daß das durch den Organismus gebildete Stoffwechselprodukt durch den Enzym-immuno-sorbent-Test (=ELISA) nachgewiesen wird.

**Europäisches Patentamt**

**EUROPÄISCHER RECHERCHENBERICHT**

**0079528**
Nummer der Anmeldung

EP 82 11 0151

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y,D P | --- DE-U-8 129 742 (HOECHST AG) <br><br> * Seite 1, Zeile 1-20 * | 1,2 | C 12 N 5/02 <br> C 12 Q 1/24 |
| Y | --- DE-A-2 945 339 (OLYMPUS OPTICAL CO.) <br> * Anspruch 1 * | 1,2 | |
| A | --- EP-A-0 015 473 (F. HOFFMANN-LA ROCHE & CO.) <br> * Ansprüche 1, 15 * | 1 | |
| A | --- DE-A-2 633 085 (OLYMPUS OPTICAL CO.) <br><br> ----- | | |

RECHERCHIERTE SACHGEBIETE (Int. Cl. 3)

C 12 N 5/00
C 12 Q 1/00

Der vorliegende Recherchenbericht wurde für alle Patentansprüche erstellt.

| Recherchenort <br> BERLIN | Abschlußdatum der Recherche <br> 02-02-1983 | Prüfer <br> SCHWARTZ K |
|---|---|---|